# EUROPEAN PATENT APPLICATION

(11) **EP 0 761 819 A1**
(43) Date of publication of application: **12.03.1997**
(21) Application number: 95114292.6
(22) Date of filing: 12.09.1995
(51) Int. Cl.: C12P 19/04, C07K 16/12, G01N 33/569, G01N 33/563, A61K 39/02, A61K 31/715, A61K 39/395, C12Q 1/04, A61K 39/104

(54) **Exopolysaccharides of burkholderia pseudomallei and burkholderia mallei**

(71) Applicant: BEHRINGWERKE Aktiengesellschaft, 35001 Marburg (DE)
(72) Inventor: Steinmetz, Ivo, Dr., D-30167 Hannover (DE); Bitter-Suermann, Dieter, Prof. Dr., D-30559 Hannover (DE)

(57) **Abstract**

Disclosed are species-specific exopolysaccharides of the bacterial species Burkholderia pseudomallei and Burkholderia mallei and methods for their preparation. Furthermore, antibodies are disclosed specifically recognising said exopolysaccharides. Additionally, the invention describes methods and kits for the diagnosis of infection by Burkholderia pseudomallei or Burkholderia mallei as well as vaccines for the immunisation against Burkholderia pseudomallei or Burkholderia mallei.

## Description

The present invention relates to species specific exopolysaccharides of the bacterial species Burkholderia pseudomallei and Burkholderia mallei and to methods for the preparation of said exopolysaccharides. Furthermore, the invention relates to antibodies recognising said exopolysaccharides. The present invention also relates to methods and kits for the diagnosis of infection by Burkholderia pseudomallei or Burkholderia mallei as well as to vaccines comprising the species specific exopolysaccharides of Burkholderia pseudomallei or Burkholderia mallei.

The gram-negative bacterial species Burkholderia pseudomallei, previously known as Pseudomonas pseudomallei (Yabuchi et al., Microbiol. Immunol. 36 (1992), 1251 to 1275) is the causative organism of melioidosis, an infectious disease of man and animals which is endemic primarily in Southeast Asia and Northern Australia. This organism lives as a saprophyte and is ubiquitous in soil and surface water, particularly in rice fields and ponds (Sanford, in "Principles and Practice of Infectious Disease", 2nd Ed., John Wiley & Sons, New York (1985), 1250 to 1254; Leelarasmee and Bovornkitti, Rev. Infect. Dis. 11 (1989), 413 to 425).
At particular risk of acquiring the infection is the rice farming population, and it is assumed that in most cases infection is caused by soil contamination of minor cuts and abrasions. The clinical manifestation of melioidosis ranges from sub-clinical to acute localised forms, acute septicemic and chronic forms (Leelarasmee and Bovornkitti, loc. cit.; Dance, Rev. Med. Microbiol. 1 (1990), 143 to 150).

Although apparently healthy individuals can be infected, septicemic melioidosis is usually associated with underlying diseases, in most cases with diabetes mellitus or chronic renal failure (Chaowagul et al., J. Infect. Dis. 159 (1989), 890 to 899), and it is known to be a major cause of morbidity and mortality in certain areas of the tropics (Dance, Clin. Microbiol. Rev. 4 (1991), 52 to 60).
In contrast to the increasing knowledge on the epidemiology of this disease, the pathogenesis of melioidosis is still poorly understood. Factors responsible for the virulence of Burkholderia pseudomallei had not yet been identified. Lipopolysaccharides, extracellular enzymes (for example, lecithinase, lipase, dermonecrotic protease) and a heatlabile exotoxine have been discussed as putative virulence factors. Due to the lack of knowledge about the pathogenesis and virulence factors of Burkholderia pseudomallei, the development of suitable treatments for melioidosis and of vaccines for immunisation has been hampered.
Furthermore, it seems likely that melioidosis is greatly underdiagnosed, especially in poor, rural areas where appropriate microbiological facilities are not available.
The currently available tests for the diagnosis of melioidosis lack specificity and selectivity as up to now, no reliable species specific antigens of Burkholderia pseudomallei have been identified. Accordingly, no antibodies specifically recognising Burkholderia pseudomallei could be provided.
Due to the inability to reliably diagnose infection by Burkholderia pseudomallei using the tests currently available, and due to the lack of vaccines for immunisation against Burkholderia pseudomallei, there is an urgent need for identifying an antigen specific for this bacterium which would allow the development of more specific and sensitive methods for diagnosing melioidosis as well as antibodies directed against Burkholderia pseudomallei and vaccines for an efficient immunisation.

The same holds true for the bacterial species Burkholderia mallei which is closely related to Burkholderia pseudomallei and which is the causative agent of glanders in horses, donkeys and mules. This bacterium is obligatory pathogen and in contrast to Burkholderia pseudomallei is not free living.
The transfer of this infection to humans normally occurs by direct contact with infected animals. Infections with Burkholderia mallei have always been rare and restricted to persons living in close contact with infected animals.
However, in humans the progress of the disease is very severe and often lethal.
As for Burkholderia pseudomallei also for Burkholderia mallei no reliable diagnosis or vaccine for immunisation is available.

Thus, the technical problem underlying the present invention is to provide species specific antigens of Burkholderia pseudomallei and Burkholderia mallei allowing reliable diagnosis of infection by these bacteria as well as the development of vaccines for immunisation and antibodies specifically recognising bacteria of the species Burkholderia pseudomallei and Burkholderia mallei.

The solution to said technical problem is achieved by providing the embodiments characterised in the claims.

Accordingly, the present invention relates to exopolysaccharides which are specifically recognised by the monoclonal antibody Mab 3015 produced by the hybridoma cell line deposited under the requirement of the Budapest Treaty on June 6, 1995 at the DSM ("Deutsche Sammlung für Microorganismen"), Braunschweig, Germany, under Accession number DSM ACC 2218. These exopolysaccharides are specifically synthesised by cells of the bacterial genus Burkholderia, preferably by cells of the species Burkholderia pseudomallei or Burkholderia mallei, and can be obtained from these cells by a method comprising the following steps:
(i) growing cells of bacteria of the genus Burkholderia;
(ii) vigorous stirring of a cell suspension of said bacterial cells, and removing bacterial cells by centrifugation;
(iii) heat-inactivation of the resulting supernatant followed by centrifugation;
(iv) subjection of the resulting supernatant to a cold 80% ethanol precipitation;
(v) dissolving of the precipitate and treatment with nucleases followed by heat-inactivation and centrifugation;
(vi) subjection of the resulting supernatant to a second ethanol precipitation; and
(vii) dissolving of the resulting precipitate followed by affinity chromatography on an affinity column containing monoclonal antibody Mab 3015 produced by hybridoma DSM ACC 2218

The bacteria cultivated in step (i) are preferably bacteria of the species Burkholderia pseudomallei or Burkholderia mallei.

For obtaining the polysaccharide of cells of Burkholderia pseudomallei in principle, any strain of Burkholderia pseudomallei can be used. The preferred source is strain NCTC 7431 of Burkholderia pseudomallei showing mucoid growth characteristics.

In a preferred embodiment, the purified exopolysaccharide of Burkholderia pseudomallei has an estimated molecular weight of more than 150 kd when determined by gel filtration on Sepharose CL-4B (Pharmacia LKB) in the presence of detergent.
Exopolysaccharides synthesised by gram-negative bacteria are generally divided into two groups (group I and II) on the basis of their different structural features and their mode of expression (Jann and Jann, in Current Topics in Microbiology and Immunology, Volume 150, K.Jann and B.Jann (Editors), Springer Verlag, Berlin (1990), 19 to 42). The high molecular weight of the exopolysaccharide synthesised by cells of Burkholderia pseudomallei, and its synthesis below temperatures of 20°C, indicate that this exopolysaccharide belongs to group I.
The findings of the present invention demonstrate that the described exopolysaccharide is constitutively produced by cells of Burkholderia pseudomallei. Despite the fact that Burkholderia pseudomallei strains may exhibit considerable variations in colony morphology ranging from the most common rough type to smooth variants, and sometimes even to mucoid growth characteristics (Dance, J. Chem. Pathol. 42 (1989), 645 to 648; Nicholls, J. Exp. Pathol. 11 (1930), 393 to 399), all tested strains originating from geographically different tropical regions, and showing different phenotypic growth characteristics, synthesise an exopolysaccharide according to the invention, namely an exopolysaccharide specifically recognised by the monoclonal antibody Mab 3015 (see Example 4).

The provision of the exopolysaccharides according to the invention is made possible by the provision of a monoclonal antibody, namely antibody Mab 3015, which specifically recognises these exopolysaccharides. Extensive testing for cross-reactivity of the antibody Mab 3015 used for the identification of said exopolysaccharides revealed that this only reacted with cells of the bacterial species Burkholderia pseudomallei and cells of the closely related species Burkholderia mallei (see Example 3).
Therefore, these exopolysaccharides represent antigens specific for the bacterial species Burkholderia pseudomallei and Burkholderia mallei, respectively and the antibody Mab 3015 allows for the identification and purification of these exopolysaccharides.

Thus, a further object of the present invention is to provide a method for the preparation of exopolysaccharides specifically synthesised by cells of the bacterial species Burkholderia pseudomallei or Burkholderia mallei, comprising the mechanical separation of the exopolysaccharide from the cell surface, followed by repetitive ethanol precipitation, and finally affinity chromatography using the monoclonal antibody Mab 3015 produced by hybridoma DSM ACC 2218.

In a preferred embodiment, said method comprises the following steps:
(i) growing cells of Burkholderia pseudomallei or Burkholderia mallei;
(ii) vigorous stirring of a cell suspension of said bacterial cells, and removing bacterial cells by centrifugation;
(iii) heat-inactivation of the resulting supernatant followed by centrifugation;
(iv) subjection of the resulting supernatant to a cold 80% ethanol precipitation;
(v) dissolving of the precipitate and treatment with nucleases followed by heat-inactivation and centrifugation;
(vi) subjection of the resulting supernatant to a second ethanol precipitation; and
(vii) dissolving of the resulting precipitate followed by affinity chromatography on an affinity column containing monoclonal antibody Mab 3015 produced by hybridoma DSM ACC 2218.

The exopolysaccharide obtainable by the method according to the invention is of high purity, and seems to be free of lipopolysaccharides, proteins and nucleic acids.
Furthermore, the purified exopolysaccharide seems to represent the native structure as no signs of breakdown can be observed and immunological detection is still possible.
Testing the reactivity of the purified exopolysaccharide of Burkholderia pseudomallei with sera of patients afflicted with melioidosis showed that a strong immunological reaction occurs (see Example 7). This indicates that the exopolysaccharide is expressed in vivo and reveals immunogenicity during natural infection.

Another object of the present invention is to provide antibodies specifically recognising the exopolysaccharides according to the invention.

A preferred embodiment is the monoclonal antibody Mab 3015 which is produced by the hybridoma deposited as DSM ACC 2218.
This antibody which recognises specifically the exopolysaccharides of cells of Burkholderia pseudomallei and of cells of Burkholderia mallei shows no cross-reactivity with other bacteria of all Pseudomonas rRNA groups. Therefore, it allows the specific detection of cells of Burkholderia pseudomallei or Burkholderia mallei and can be used for the preparation of the species specific exopolysaccharides. These can be used for the production of antibodies specifically recognising each exopolysaccharide.

Therefore, the invention also relates to antibodies specifically recognising an exopolysaccharide synthesised by cells of the bacterial species Burkholderia pseudomallei which is specifically recognised by monoclonal antibody Mab 3015 produced by the hybridoma which is deposited as DSM ACC2218.

Furthermore, the invention relates to antibodies specifically recognising an exopolysaccharide synthesised by cells of the bacterial species Burkholderia mallei which is recognised by monoclonal antibody Mab 3015 produced by hybridoma DSM ACC 2218.

In a preferred embodiment of the present invention, the antibody specifically recognising an exopolysaccharide synthesised by cells of Burkholderia pseudomallei or Burkholderia mallei is a monoclonal antibody, preferably of the IgG1-type which is derived from any mammal, for example, a rat, mouse, goat, and the like, by conventional techniques as described, for example, in Köhler and Milstein, Nature 256 (1975), 495; Galfrè, Meth. Enzymol. 73 (1981), 3, or Harlow and Lane, Antibodies, A Laboratory Manual (1988), Cold Spring Harbor. Preferably, the monoclonal antibody recognising the exopolysaccharide of cells of Burkholderia pseudomallei or Burkholderia mallei is derived from mice.

In another preferred embodiment, the antibody according to the invention is a polyclonal antibody. The preparation of polyclonal antibodies is well known in the art and is described, for example, by Harlow and Lane (loc. cit.).

In yet another preferred embodiment, the antibody according to the invention is a chimeric, semisynthetic or synthetic antibody.

In another embodiment, the present invention relates to fragments of an antibody specifically recognising the exopolysaccharide of cells of Burkholderia pseudomallei or the exopolysaccharide of cells of Burkholderia mallei. These fragments typically have the binding specificities of said antibodies of the invention. They can be produced by conventional techniques as described in Bagulawski et al., J. Immunol. Meth. 120 (1988), 51, and Weir (ed.), Handbook of Experimental Immunology, Blackwell, Edinburgh (1986).
In a preferred embodiment, these fragments are Fv, Fab or F(ab)2 fragments.

In a further preferred embodiment, the invention relates to chemically modified derivatives of said monoclonal, polyclonal, chimeric, semi-synthetic or synthetic antibodies or antibody fragments.

Another object of the present invention is to provide continuous and stable cell lines producing an antibody, antibody fragment or a derivative thereof according to the invention, which specifically recognises the exopolysaccharide of cells of the bacterial species Burkholderia pseudomallei or the exopolysaccharide of Burkholderia mallei.

In a preferred embodiment, said cell line is a hybridoma.
The preparation of hybridoma cell lines is well known in the art, and is described, for example, in Harlow and Lane (loc. cit.). Particularly preferred is the hybridoma that produces the monoclonal antibody Mab 3015, and which has deposited as DSM ACC 2218.

A further object of the present invention is to provide a method for producing antibodies, fragments or derivatives thereof which specifically recognise the exopolysaccharide of cells of Burkholderia pseudomallei or Burkholderia mallei.
In one embodiment, said method comprises culturing of an antibody producing cell line according to the invention, and isolating the antibody, antibody fragment or derivative.

The provision of antibodies specifically recognising the exopolysaccharide of Burkholderia pseudomallei and/or the exopolysaccharide of Burkholderia mallei, allows for the development of methods to detect infections of an individual with one of these bacteria. Thus, a further object of the present invention is to provide methods for the in vitro diagnosis of infection by Burkholderia pseudomallei, wherein samples of body fluids or tissues are tested for their reactivity with antibodies or fragments or derivatives thereof which specifically recognise the exopolysaccharide of cells of Burkholderia pseudomallei. The invention also relates to methods for the in vitro diagnosis of infection by Burkholderia mallei, wherein samples of body fluids or tissues are tested for their reactivity with antibodies or fragments or derivatives thereof which specifically recognise the exopolysaccharide of cells of Burkholderia mallei.
In these methods antibodies can also be used which recognise the exopolysaccharide of both Burkholderia species, such as antibody Mab 3015. Since infections by Burkholderia pseudomallei and Burkholderia mallei in humans are treated by the same methods, it is in the first place only important to determine whether a patient is infected by one of these bacteria. The determination of the specific species can be carried out with the above described species specific antibodies.

Methods for detecting the reactivity of an antibody with an antigen are well known in the art, and include, for example, Radio Immuno Assay (RIA), Enzyme Linked Immuno Solvent Assay (ELISA), Western Blotting and the like (see, for example, "Immuno Assay: A Practical Guide", Chan and Perlstein (Editors) 1987). In a preferred embodiment, the sample is blood, serum or plasma.

A further object of the invention is to provide kits for use in in vitro diagnosis of infection by Burkholderia pseudomallei, which comprises an antibody or fragment or derivatives thereof according to the invention which recognises the exopolysaccharide of cells of Burkholderia pseudomallei. The invention also relates to kits for use in in vitro diagnosis of infection by Burkholderia mallei, which comprises an antibody or fragment or derivatives thereof according to the invention which recognises the exopolysaccharide of cells of Burkholderia mallei. Such a kit may optionally comprise further reagents conventionally used in immuno diagnostic methods.
Again, the described kits can also comprise antibodies which recognise the exopolysaccharides of both Burkholderia species like monoclonal antibody Mab 3015.

Another object of the present invention is to provide methods for the in vitro detection of antibodies directed against cells of the bacterial species Burkholderia pseudomallei in body fluids or tissue, which comprises testing samples of body fluids or tissue for their reactivity with an exopolysaccharide according to the invention specifically synthesised by cells of Burkholderia pseudomallei.
Similarly, the present invention relates to methods for the detection of antibodies directed against cells of Burkholderia mallei in body fluids or tissue, which comprises testing samples of body fluids or tissue for their reactivity with an exopolysaccharide according to the invention specifically synthesised by cells of Burkholderia mallei. In a preferred embodiment, the sample used in these methods is blood. Methods for detecting the reactivity of an antibody with an antigen are well known in the art, and include, for example, Radio Immuno Assay (RIA), Enzyme Linked Immuno Solvent Assay (ELISA), Western Blotting and the like (see, for example, "Immuno Assay: A Practical Guide", loc. cit.).
In order to detect antibodies directed against the exopolysaccharide synthesised by cells of Burkholderia pseudomallei or Burkholderia mallei, respectively, intact bacteria lysed or heat treated bacteria may be used.
Preferably the purified exopolysaccharide according to the invention, is used.

Still a further object of the present invention is a kit for the in vitro detection of antibodies directed against cells of the bacterial species Burkholderia pseudomallei, comprising an exopolysaccharide according to the invention, which is specifically synthesised by cells of Burkholderia pseudomallei. The invention also relates to kits for the in vitro detection of antibodies directed against cells of Burkholderia mallei, comprising an exopolysaccharide according to the invention which is specifically synthesised by cells of Burkholderia mallei. Such a kit may optionally comprise further reagents conventionally used in immuno diagnostic methods.

Finally, a further object of the present invention is to provide vaccines for the immunisation against infection by Burkholderia pseudomallei, which comprises an exopolysaccharide synthesised by cells of Burkholderia pseudomallei according to the invention.

The invention also relates to vaccines for immunisation against infection by Burkholderia mallei, which comprises an exopolysaccharide synthesised by cells of Burkholderia mallei according to the invention. According to the invention it is also possible to prepare vaccines which comprise exopolysaccharides of both Burkholderia species and therefore, confer protection against infection by both.

For this purpose, said exopolysaccharides can be used in a soluble form and formulated as a vaccine in analogy to the commercially available vaccine Pneumovax, 23 against Pneumococcus species. The exopolysaccharide may be dissolved in saline, PBS (Phosphate Buffered Saline) or other suitable solvents conventionally used in the preparation of pharmaceuticals. Optionally, further agents could be added, such as preservatives (phenol, sodium timerfonate, inter alia), stabiliser (for example, sugars, amino acids, proteins), or adjuvants as described in summary in Vogel and Powell ("A Compendium of Vaccine Adjuvants and Excipients" in: Vaccine Design, Powell and Newman, Editors, Plenum Publishing Corporation, New York).
Furthermore, the exopolysaccharide may be adsorbed to a carrier such as aluminium hydroxide, aluminium oxide, aluminium phosphate or calcium phosphate, or salts of zinc.

In a preferred embodiment, the vaccine further comprises one or more other antigens, preferably polysaccharides, such as those from Pneumococcus species, Meningococcus species, Haemophilus species, and, in a particularly preferred embodiment, the antigenic polysaccharide of Pseudomonas aeruginosa.

In another embodiment, it is possible to prepare a vaccine in which the exopolysaccharide is present as a conjugate with an immunogenic carrier. Thereby, the immunogenicity can be increased, and it is possible to induce a T-cell dependent booster effect as developed, for example, for the vaccine comprising as antigen the polyribosyl-ribitol-phosphate of Haemophilus influencae B. Examples of carrier proteins that can be used in this context are the tetanus toxoid, the diphteria toxoid, the pertussis toxoid, or the outer membrane complex of Neisseria meningitis (for a review, see Hungerer et al., Allg. Med. 70 (1994), 585 to 589). Other suitable carriers are antigens derived from Pseudomonas, as, for example, the detoxified toxine A.

The coupling of the exopolysaccharide to the carrier may, for example, be achieved by chemical means known in the art.
Furthermore, it is possible to use a linker for the conjugation of the exopolysaccharide with a carrier.

In a preferred embodiment, the exopolysaccharide present in the vaccine may be further modified.

An object of the present invention is also to provide vaccines comprising antibodies specifically recognizing an exopolysaccharide synthesized by cells of the bacterial species Burkholderia pseudomallei and/or antibodies specifically recognising an exopolysaccharide synthesized by cells of the bacterial species Burkholderia mallei.
These vaccines can also comprise antibodies which recognise the exopolysaccharides of both Burkholderia species, like monoclonal antibody Mab 3015.

### LEGENDS TO THE FIGURES :

- **Figure 1:**: Immunoblot with Mab 3015 of SDS-page (3 - 10%) with affinity purified exopolysaccharide and whole bacterial cells of B. pseudomallei NCTC 7431.
Lanes:
   1, exopolysaccharide;
   2, whole cells;
   3, P. aeruginosa 492 alginate as a negative control.
- **Figure 2:**: Immunoelectron microscopic localisation of an exopolysaccharide of B. pseudomallei NCTC 7431 using postembedding labelling with Mab 3015.
**A**, bacterial cells without pretreatment with the specific Mab 3015 exhibit some labelling on the cell periphery, ie the outer membrane region;
**B** and **C**, bacteria preincubated with Mab 3015 show preservation of the capsular structures (arrows).
   The intensity of the label is higher compared to nontreated cells (A). The exopolysaccharide is mostly located in the capsular structures and not in the outer membrane region of the cell;
**D**, sections of pretreated bacteria (Mab 3015) were incubated with a control Mab IgG1 (2454) followed by protein A-gold complexes, only very few gold particles (arrow heads) are detectable.
   The large granules within the cytoplasma most likely represent poly-β-hydroxybutyrate accumulation which is characteristic for Burkholderia species (Palleroni, in "The Prokaryotes", Barlow, Truper, Dworkin, Harder, Schleifer (ed), Vol. 3, Second Edition, Springer Verlag (1992), 3086 to 3103). G, gold-particle, OM, outer membrane; bars represent 100 nm in A-C and 0.5 µm in D.

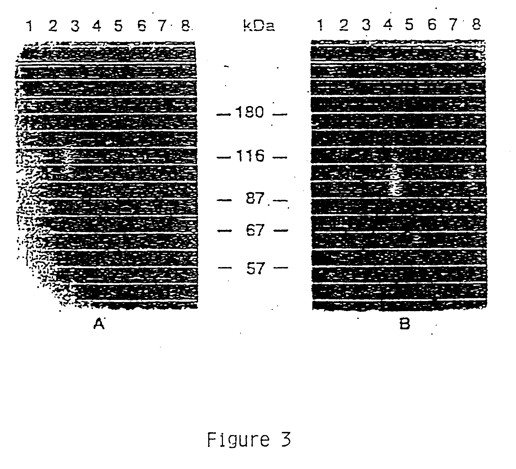

- **Figure 3:**: Representative immunoblot with Mab 3015 of SDS-Page (7.5%) with different B. pseudomallei strains and non-B. pseudomallei strains.
**A**, lanes:
   1, B. pseudomallei NCTC 4845;
   2, B. pseudomallei NCTC 4846;
   3, B. pseudomallei NCTC 6700;
   4, B. pseudomallei NCTC 7383;
   5, B. pseudomallei NCTC 8016;
   6, B. pseudomallei NCTC 10274;
   7, B. pseudomallei NCTC 10276;
   8, B. pseudomallei NCTC 8708;
**B**, lanes:
   1, P. aeruginosa LMG 1242 T;
   2, P. alcalingenes LMG 1224 t1;
   3, B. cepacia LMG 1222 T;
   4, B. pickettii LMG 5942 T;
   5, B. solanacearum LMG 2299 T;
   6, P. vesicularis LMG 2350;
   7, S. maltophilia ATCC 17406;
   8, P. mendocina LMG 1223 T.

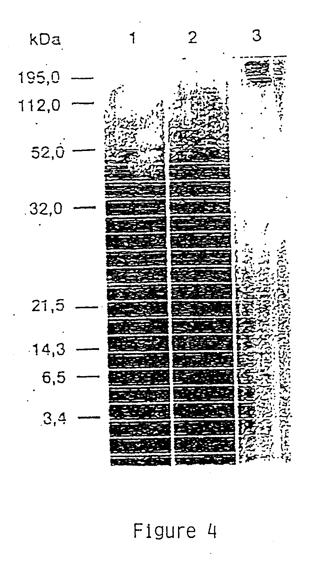

- **Figure 4:**: Silver-staining of SDS-page (15%) with affinity purified exopolysaccharide of B. pseudomallei NCTC 7431 and bacterial extracts of the same strain after proteinase K digestion for the detection of LPS.
Lanes:
   1, exopolysaccharide;
   2, bacterial extract;
   3, immunoblot with Mab 3015 with the same sample and SDS-page as in lane 2.

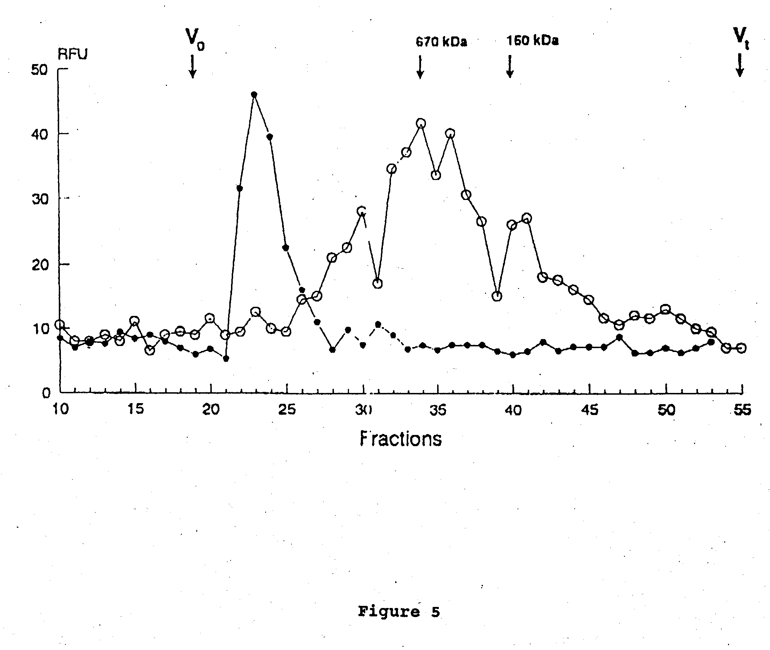

- **Figure 5:**: Gel-filtration chromatography of the affinity purified exopolysaccharide of B. pseudomallei NCTC 7431 on a column (1.6 cm by 50 cm) of Sepharose CL-4B. The elution profile as determined by ELISA (RFU) is shown. • shows the profile when the eluant was 0.1 M PBS, pH 7.4 containing 0.1 M EDTA. 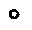 shows the profile when the exopolysaccharide was dissolved in 3% deoxycholate and incubated for 1 hour at 56°C prior to elution in PBS containing 0.25% deoxycholate. The different dextrans standards are indicated together with the void volume (Vₒ) and the total volume (Vₜ).

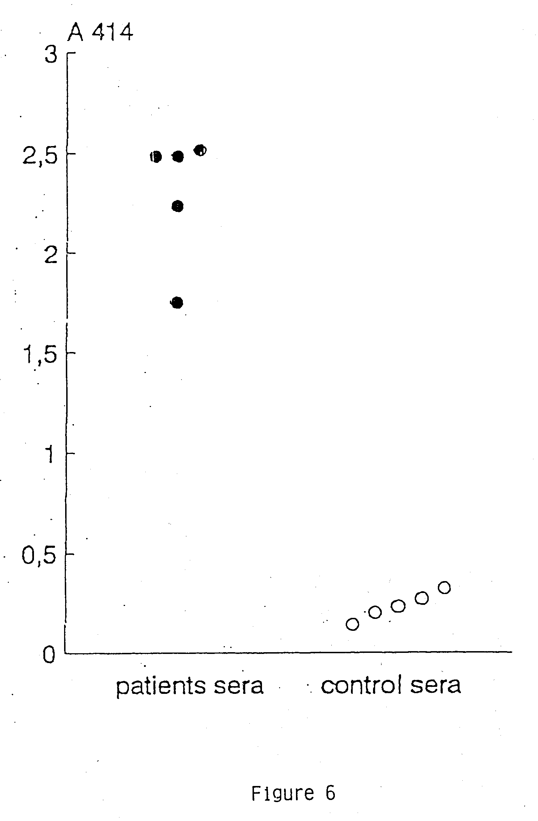

- **Figure 6:**: The reactivity of sera (1:2.500 dilution) from five melioidosis patients and five control sera with the affinity purified exopolysaccharide in an ELISA-assay is shown.

### BACTERIAL STRAINS USED IN THE EXAMPLES AND CULTIVATION CONDITIONS

Tables 1 and 2 in Examples 3 and 4 list all of the bacterial strains used in this invention. Strains designated as LMG were type strains from the Belgian Collection of Culture Strains (Universiteit Gent, Laboratorium voor Microbiologie, Gent, Belgium). ATCC and NCTC strains were either from the American Type Culture Collection or the National Collection of Type Cultures, respectively. Bacteria were grown at 37°C on sheep blood agar (SBA) unless otherwise stated.

The following examples illustrate the invention.

### EXAMPLE 1: Production of monoclonal antibodies specifically recognising cells of the bacterial species Burkholderia pseudomallei

Six-week old female BALB/c mice (Zentralinstitut fur Versuchstierkunde, Hannover, FRG) were immunised once a week intraperitoneally over a period of 4 weeks with 2 x 10⁸ heat inactivated (80°C for 1 hour in buffer A; 0.01 M potassium phosphate buffer made isotonic with saline, pH 7.5) B. pseudomallei NCTC 7431 cells. The first injection was given with complete Freund's adjuvant. The second injection was given with incomplete Freund's adjuvant (Difco). The following injections were given with physiological saline. At the end of the immunisation regime, the mice were splenectomised and the spleen cells were fused with X63-AgB 653 myeloma cells as described in Peters et al. (Infect. Immun. 50 (1985), 459 to 466). The culture supernatant fluids of growing clones were screened by ELISA with B. pseudomallei NCTC 7431 whole cells as an antigen (see below).
The resulting hybridomas were cloned by limiting dilution. For the production of ascites fluid, hybridoma cells were injected intraperitoneally into Pristane-pretreated Balb/c mice.

The detection of monoclonal antibodies against B. pseudomallei was performed by ELISA assays in U-shaped wells of non-flexible polystyrol microtiter plates (Greiner, Nürtingen, FRG). Plates were coated with heat treated B. pseudomallei NCTC 7431 grown on SBA plates as follows. Cells were washed with physiological saline and adjusted in buffer A to 2 x 10⁸ cells per ml. 20 µl of the suspension was added per well for 2 hours at room temperature. Plates were washed twice with 200 µl buffer A and then incubated with buffer A-BSA (buffer A containing 1% (wt/vol) bovine serum albumin, Merck, Darmstadt, FRG) for 30 minutes. Hybridoma culture supernatant fluids (20 µl) and culture medium as a negative control were then added and plates were incubated for 2 hours then washed extensively with buffer A prior to addition of 10 µl biotin-labelled goat-anti-mouse immunoglobulin (Jackson Immunoresearch Laboratories, USA, diluted 1:2000 in buffer A-BSA) for 30 minutes. Plates were then washed with buffer A, and 10 µl of streptavidin coupled to β-galactosidase (Boehringer GmbH, Mannheim, FRG) diluted 1:2000 in buffer A-BSA was added and incubated for 30 minutes. The microtiter plates were then washed twice with buffer A and once with 0.01 M sodium-phosphate-buffer, pH 6.9. Substrate solution (20 µl, 0.1 mM 4-methylumbelliferyl-β-D-galactopyranasoside, Boehringer Mannheim, FRG, in 0.01 M sodium-phosphate-buffer, pH 6.9) was added to each well and the fluorescent product was measured in a Titertek Fluoroskan 2 (Labsystems, Helsinki, Finland) as Relative Fluorescence units (RFU), excitation 366 nm, emission 460 nm.

Several hybridoma clones producing monoclonal antibodies that reacted strongly with whole cells of B. pseudomallei NCTC 7431 in ELISA assays were identified. The antibody Mab 3015 (IgG1 isotype) produced by the hybridoma cell line, which is deposited as DSM ACC 2218, was selected for further studies. This antibody was tested against whole cell lysats of B. pseudomallei in an immunoblot following SDS polyacrylamid gel electrophoresis.

For this purpose, bacteria grown on agar plates were washed twice with physiological saline and adjusted to a concentration of 5 x 10⁹ cells per ml. After centrifugation in an Eppendorf Biofuge at 10000 x g for 5 minutes, the pellet from 1 ml of the bacterial suspension was suspended in 1 ml of sample buffer (0.0625 M Trishydrochloride, pH 6.8, 5% 2-mercaptoethanol, 2% SDS, 12,5% glycerol), and heated for 10 minutes at 100°C. A sample of 20 µl (equivalent to 10⁸ bacteria) was applied for each lane of the gel. Molecular weights were estimated by comparison with a prestained SDS marker kit (MW-SDS-Blue, Sigma). SDS slab gel electrophoresis was performed as previously described in Peters et al. (Infect. Immun. 50 (1985), 459 to 466). When purified exopolysaccharide or bacterial extracts were examined, 8 µg were applied for each lane of the gel. The blotting procedure was done by previously described methods (Peters et al. (1985) loc. cit.) with the minor modification of using 200 V for 3 hours. The nitrocellulose sheet was saturated with 2% (wt/vol) instant dried milk in buffer A overnight. The monoclonal antibody Mab 3015 was purified by affinity chromatography on protein A-sepharose (Pharmacia, Upsala, Sweden) and diluted 1:2000 in 2% instant dried milk-buffer A. Peroxidase-conjugated goat anti-mouse immunoglobulin (Jackson Immunoresearch Laboratories, USA, 1:2000 in 2% dried-milk-buffer A) was used for final development. The substrate was 4-chloro-1-naphtol (Sigma).

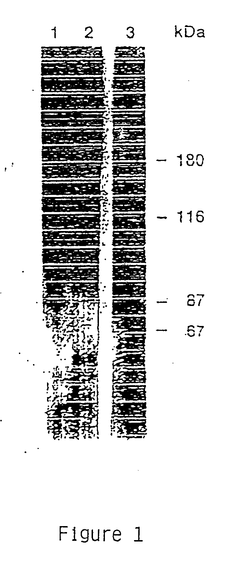

In the immunoblot, the monoclonal antibody Mab 3015 revealed reactivity with a diffuse band in a high molecular mass range (Figure 1). This reactivity could not be eliminated by incubating the immunoblot with proteinase K. These data indicated that the epitope recognised by the monoclonal antibody Mab 3015 was a surface located carbohydrate. As the strain used for immunisation, namely strain NCTC 7431, produces a mucoid substance, it was hypothesised that this substance may be the epitope recognised by Mab 3015. To verify this, immunoelectron microscopy experiments were performed.

### EXAMPLE 2: Identification of the antigen recognised by monoclonal antibody Mab 3015 as an exopolysaccharide

Immunoelectron microscopy was performed as follows:

Postembedding labelling: For the preservation of capsular structures, the bacteria were pretreated (a) with 1:50 diluted ascites of the specific Mab 3015 IgG1 for 45 minutes at room temperature, (b) with Mab 2454 IgG1 specific for the H. influencae type B polysaccharide for 45 minutes at room temperature as a control, and (c) without any pretreatment. After three washing steps with PBS (0.1 M sodium phosphate buffer, 0.15 M NaCl, pH 6.9) the bacteria were fixed with 0.5% formaldehyde and 0.2% glutaraldehyde (final concentrations) in PBS for 1 hour on ice. After three washes with PBS containing 10 mM glycine to block free aldehyde groups, the cells were embedded by progressively lowering the temperature using Lowicryl K4M resin (Roth et al., J. Histochem. Cytochem. 29 (1981), 663 to 669). The following modifications were made to this method: (a) after dehydration in 10% ethanol, the samples were treated with 0.5% uranyl acetate in 10% ethanol for 1 hour on ice, (b) the infiltration step with 1 part ethanol and 1 part K4M resin was performed overnight, (c) the infiltration step with 1 part ethanol and 2 parts K4M resin lasted for 12 hours, (d) infiltration with pure K4M resin lasted for 2 days. After polymerisation of the samples for 2 days at -35°C, samples were trimmed and polymerised for another day at room temperature. Ultrathin sections were incubated with Mab 3015 or the control Mab (200 µg IgG1/ml) overnight at 4°C and washed with PBS, followed by incubation of the sections with protein A-gold complexes (10 nm in size, concentration giving an A₅₂₀ of 0.02). Sections were subsequently rinsed with PBS containing 0.01% Tween 20 and then with distiled water.
After being air-dried, the sections were counterstained with 4% aqueous uranyl acetate, pH 4.5, for 5 minutes. Samples were examined with a Zeiss electron microscope EM 910 at an acceleration voltage of 80 kV at calibrated magnifications.

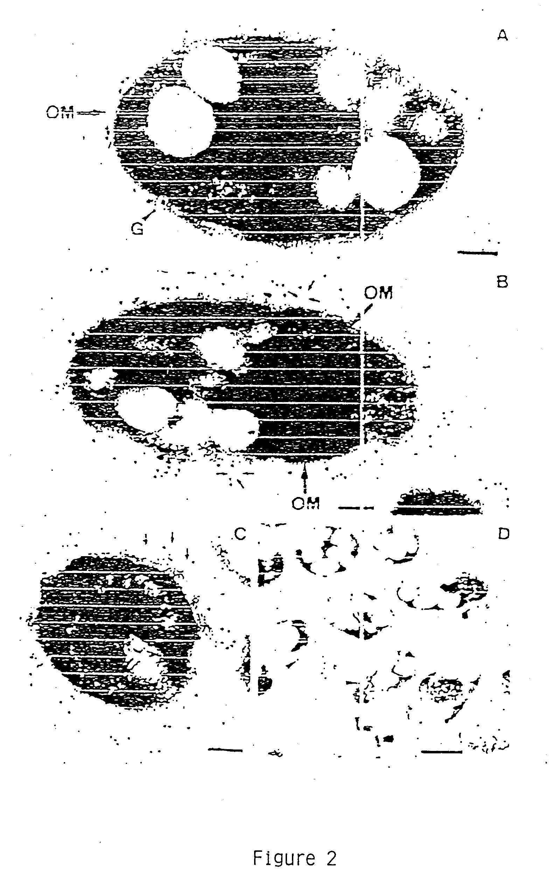

The results of immunoelectron microscopy experiments using different experimental conditions are summarised in Figure 2.
Figures 2b and 2c demonstrate reactivity of Mab 3015 with an exopolysaccharide of capsule like appearance in the immunising strain. These results were obtained by incubating the cells with Mab 3015 before fixation in order to crosslink and to preserve the capsule structure. In Figure 2a, cells were not pretreated in this way. Although cell surface bound antibody could be detected, the capsule structure was much less preserved. This is probably due to the collapse of the structure during the fixation procedure.

### EXAMPLE 3: Determination of the specificity of the monoclonal antibody Mab 3015

In order to demonstrate the specificity of Mab 3015, extensive testing for cross-reactivity by ELISA and immunoblot was performed. Strains from all Pseudomonas rRNA groups were tested together with strains of the former rRNA group II, which have now been transferred to the novel genus Burkholderia (Yabuchi et al., Microbiol. Immunol. 36 (1992), 1251 to 1275).
ELISA assays and immunoblot were performed as described in Example 1.

The tested strains are listed in the following table.

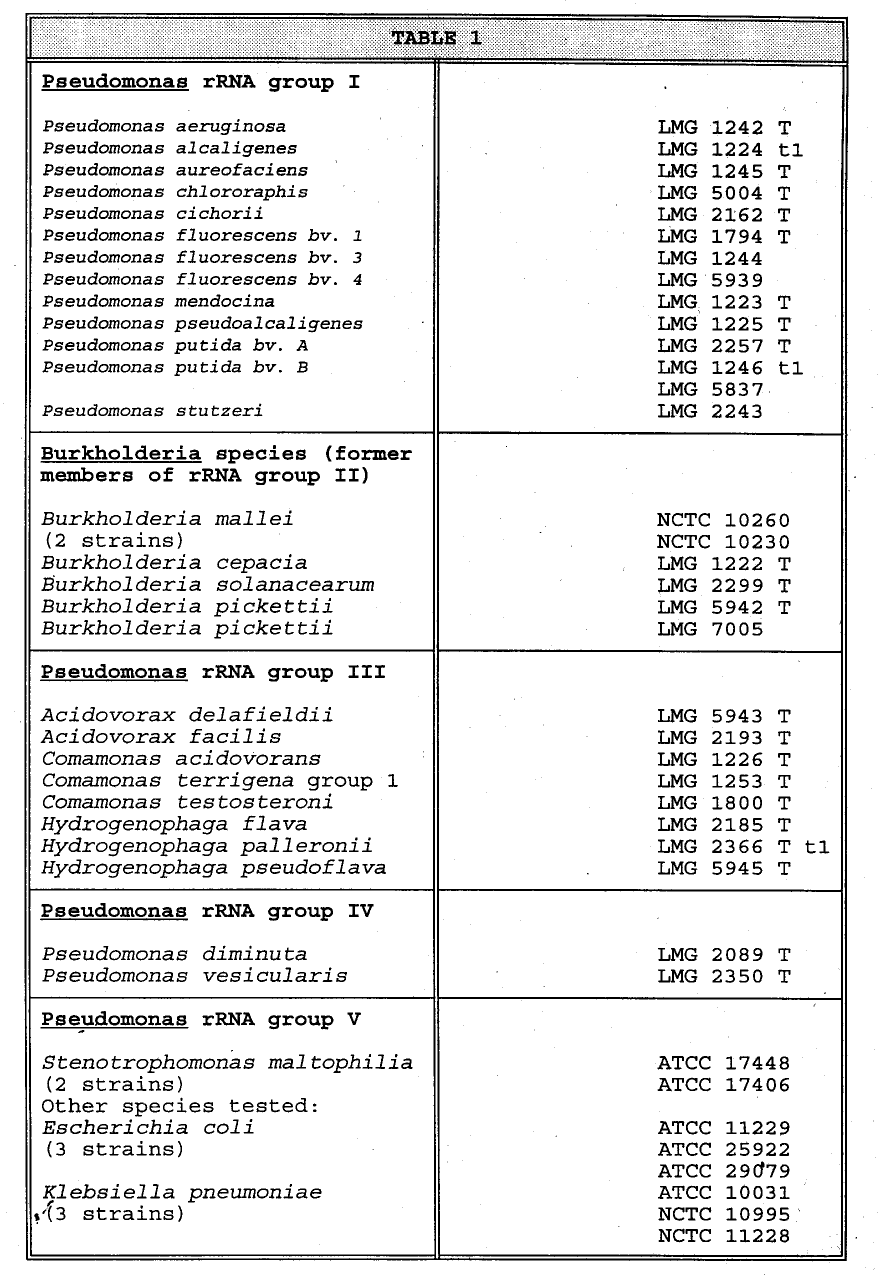

No cross-reactions could be detected with the exception of the closely related B. mallei. The result of an immunoblot is shown in Figure 3.

### EXAMPLE 4: Determination of the expression of the exopolysaccharide among B. pseudomallei strains

To investigate the distribution of the exopolysaccharide among the B. pseudomallei species, 12 different strains from different geographical regions, most of them showing a rough-type growth pattern, were tested with Mab 3015.

The tested strains are listed in the following table.

**TABLE 2**

| **B.PSEUDOMALLEI STRAINS TESTED** | | | **ORIGIN** |
|---|---|---|---|
| B.pseudomallei | NCTC | 4845 | Singapore |
| B.pseudomallei | NCTC | 1688 | Malaysia |
| B.pseudomallei | NCTC | 4846 | Singapore |
| B.pseudomallei | NCTC | 6700 | Malaysia |
| B.pseudomallei | NCTC | 7383 | Burma |
| B.pseudomallei | NCTC | 7431 | Unknown |
| B.pseudomallei | NCTC | 8016 | Australia |
| B.pseudomallei | NCTC | 10274 | Malaysia |
| B.pseudomallei | NCTC | 10276 | Bangladesh |
| B.pseudomallei | NCTC | 8707 | Singapore |
| B.pseudomallei | NCTC | 8708 | Singapore |
| B.pseudomallei | NCTC | 11642 | Unknown |

The listed strains were tested for their reactivity with the monoclonal antibody Mab 3015 in ELISA assays as well as in immunoblot experiments as described in Example 1.

All strains were positive in the ELISA and immunoblot demonstrating a constitutive expression of the exopolysaccharide independent of the growth appearance and geographical origin. A representative immunoblot with different strains is shown in Figure 3A. It was also investigated whether the expression of the exopolysaccharide is dependent on the growth temperature. Different B. pseudomallei strains were grown at 15°C, and subsequent testing in an immunoblot and ELISA revealed the same reactivity pattern with Mab 3015 as with bacteria grown at 37°.

### EXAMPLE 5: Purification of Burkholderia pseudomallei exopolysaccharide

The B. pseudomallei strain NCTC 7431 used for immunisation was also used to establish a purification protocol of the exopolysaccharide. This strain produced large amounts of a mucoid substance on the modified Ashdown agar, and, therefore, the purification procedure was commenced with bacteria grown under these conditions. The first step involved the mechanical separation of the mucoid substance from the cells by vigorous stirring of a cell suspension and subsequent centrifugation to remove bacterial cells. The resulting supernatant was heat inactivated, and after centrifugation, the supernatant was subjected to a cold 80% ethanol precipitation. The dissolved precipitate was treated with nucleases to remove nucleic acids. After heat inactivation of the enzymes and centrifugation, the supernatant was used for a second ethanol precipitation.
During the final purification step, the dissolved precipitate was used for affinity chromatography with Mab 3015, which was bound to protein A sepharose. Elution of the bound exopolysaccharide was performed under alkaline conditions.

It detail, the purification of the exopolysaccharide was performed in the following way:
Mucoid strain NCTC 7431 was grown to confluence on modified Ashdown agar plates (Dance, J. Clin. Phathol. 42 (1989), 645 to 648) for 72 hours at 37°C. Colonies were harvested with 20 ml of PBS (0.01 M sodium phosphate buffer made isotonic with saline, pH 7.4) per plate. The collected mucoid material was pooled and stirred vigorously on a magnetic stirrer for 1 hour to separate the mucoid layer from the cells and to get a uniform solution. The solution was then centrifuged for 4 hours at 20000 x g at 4°C to remove the whole bacteria. The supernatant was heated for 30 minutes at 80°C to kill the left over viable bacteria and centrifuged for 30 minutes at 20000 x g at 4°C. The supernatant was precipitated by adding cold absolute ethanol to a final concentration of 80% (vol/vol) for 1 to 2 hours at -20°C.
The precipitate was collected by centrifugation for 30 minutes at 3000 x g at 4°C and washed once in 80% ethanol for 30 minutes, and again centrifuged for 30 minutes. This washing step was repeated once with 96% ethanol. The precipitate was then dissolved in 0.01 M PBS (containing 10 mM MgCl₂ and 1 mM CaCl₂) and RNase A (100 µg/ml, Sigma) and DNase 1 (100 µg/ml, Sigma) were added for 2.5 hours at 37°C to remove the contaminating RNA and DNA. After incubation enzymes were inactivated and denatured by heating for 30 minutes at 80°C, followed by centrifugation for 30 minutes at 20000 x g at 4°C. The supernatant was used for a second precipitation procedure with 80% ethanol, as above, and after centrifugation, the precipitate was dissolved in 2 ml aqua dest. This material was then used for affinity chromatography. Mab 3015 IgG1 was coupled to protein A sepharose (Pharmacia) using dimethylpimelimidate (DMP) as a coupling reagent as described (Harlow and Lane, Antibodies: A Laboratory Manual, Cold Spring Harbor Laboratory, Cold Spring Harbor, New York (1988), 555 to 562). For elution of the polysaccharide from the column 100 mM triethylamine, pH 11,5 was used. Fractions were collected and immediately neutralised using acetic acid. Each fraction was tested in the ELISA for the presence of exopolysaccharide using Mab 3015, and positive factions were pooled, dialysed against aqua dest overnight, and, finally, lyophilised.

The ELISA assay was performed as described in Example 1 with two modifications. Plates were coated with 20 µl of each fraction eluted from the column as antigen, and the monoclonal antibody Mab 3015 was used as the detecting antibody.

The purity of the exopolysaccharide preparation for protein and lipopolysaccharide contamination was analysed by SDS page followed by a highly sensitive silver staining procedure for the detection of proteins (Switzer et al., Anal. Biochem. 98 (1979), 231 to 237) and lipopolysaccharides (LPS) (Tsai and Frasch, Anal. Biochem. 119 (1982), 115 to 119). For a more sensitive detection of LPS, a quantitative limulus amoebocyte lysate assay was performed. A ₂₆₀ was measured for DNA and RNA detection.

The exopolysaccharide obtained after affinity chromatography was of high purity and gave an orange-yellow colour in the phenol-sulphuric acid assay. The preparation did give a strong signal in the immunoblot using Mab 3015 (Figure 1).
The reaction of the purified preparation was the same when compared to whole cell lysates, and this shows that no markable degradation occurred during the purification process (Figure 1). Using highly sensitive silver staining, neither LPS (Figure 4, lane 1) nor protein (not shown) contaminations could be detected. As a control, LPS silver staining of bacterial extracts of B. pseudomallei after proteinase K digestion was performed. For this purpose, cells of B. pseudomallei NCTC 7431 were harvested and killed by 0.5% phenol followed by washing steps with ethanol, twice with acetone, and once with diethylether. The bacteria were dried and then digested with RNase A (Sigma, 100 µg/ml), DNase 1 (Sigma, 100 µg/ml) and proteinase K (Sigma, 100 µg/ml).
After extensive dialysis, the extract was lyophilised. The extracts were used for SDS gel-electrophoresis. The results revealed that the LPS had a much lower molecular mass (Figure 4, lane 2) compared to the exopolysaccharide detected by the immunoblot of the same preparation, which hardly entered the 15% running gel (Figure 4, lane 3).
In a further analysis, LPS contamination of the affinity-purified exopolysaccharide was calculated to be 0.0025% by Limulus test. Nucleic acids were not detectable by spectroscopy.

### EXAMPLE 6: Determination of the molecular mass of the exopolysaccharide

The molecular mass of the purified exopolysaccharide was determined on a column of Sepharose CL-4B, which is designed to separate large molecules by gel filtration.

The column (1.6 cm by 50 cm) was equilibrated in 0.01 M PBS, pH 7.4 and eluted with the same buffer at a flow rate of 24 ml/h. Fractions of 1.8 ml were collected. Void volume and total volume were determined with blue dextran 2000 (Pharmacia) and acetone, respectively. Dextrans of 670 kD and 150 kD (Fluka, Biochemia, FRG) were used as standards and detected by the phenol-sulphuric acid assay (Dubois et al., Anal, Chem. 28 (1956), 350 to 356). 20 µg of purified polysaccharide dissolved in 500 µl PBS were applied to the column. Fractions were tested for the presence of the polysaccharide in the ELISA as described in Example 1 with two modifications. Plates were coated with 20 µl of each fraction eluted from the column as antigen, and the monoclonal antibody Mab 3015 was used as the detecting antibody.
First the elution was performed with PBS containing EDTA and the exopolysaccharide eluted in a single peak after the void volume and was found to be in a molecular mass range of >500 kd compared to dextran standards (Figure 5).

To obtain an elution pattern under conditions which exclude possible aggregation or micelle formation, the exopolysaccharide was then eluted in the presence of deoxycholate. As shown in Figure 5, the peak observed without deoxycholate was completely lost and the main peak of the exopolysaccharide was found in a molecular mass range of >150kD compared to dextran standards. These results suggest that the exopolysaccharide is able to form aggregates which can be dissociated by an anionic detergent.

### EXAMPLE 7: Reactivity of human patient sera with the affinity-purified exopolysaccharide

In order to analyse whether the identified exopolysaccharide is immunogenic in humans, sera from patients with septicemic melioidosis were tested against the affinity-purified exopolysaccharide in an ELISA.
The ELISA used was, in principle, identical to the ELISA described in Example 1, but a different developing system was used.
Plates were coated with 20 µl affinity-purified exopolysaccharide (50 µg per ml, diluted in buffer A) for 2 hours. After incubation of sera (20 µl, diluted in buffer A-BSA) for 2 hours, 10 µl peroxidase-labelled goat-anti-human immunoglobulin (Dianova, FRG, diluted 1:3000 in buffer A-BSA) was added for 30 minutes. As a substrate solution, 20 µl 2.7% 2,2'-azino[di(3-ethylbenzthiazolinesulfonic acid)] (Sigma, FRG) in potassium phosphate buffer, pH 6.0, containing 0.0025% H₂O₂ was used, and A₄₁₄ was measured in a Titertek Multiscan MC (Flow Laboratories, FRG).
Sera from four patients which acquired infection in Thailand, and one patient which acquired infection in Malaysia, were tested.
The strong reactivity of the tested sera (Figure 6) demonstrates the production of antibodies against this structure in humans and also indicate that the exopolysaccharide is expressed in vivo. In the immunoblot, the reactivity pattern was the same as the pattern found with Mab 3015 (not shown).

## Claims

1. An exopolysaccharide which is specifically recognised by monoclonal antibody Mab 3015 produced by the hybridoma DSM ACC 2218.

2. The exopolysaccharide of claim 1 which is synthesised by bacteria of the genus Burkholderia.

3. An exopolysaccharide synthesised by bacteria of the genus Burkholderia obtainable by a method comprising the following steps:
(i) growing cells of the genus Burkholderia;
(ii) vigorous stirring of a cell suspension of said bacterial cells and removing bacterial cells by centrifugation;
(iii) heat-inactivation of the resulting supernatant followed by centrifugation;
(iv) subjection of the resulting supernatant to a cold 80% ethanol precipitation;
(v) dissolving of the precipitate and treatment with nucleases followed by heat-inactivation and centrifugation;
(vi) subjection of the resulting supernatant to a second ethanol precipitation; and
(vii) dissolving of the resulting precipitate followed by affinity chromatography on an affinity column containing monoclonal antibody Mab 3015 produced by hybridoma DSM ACC 2218.

4. The exopolysaccharide of any one of claims 1 to 3, which is synthesised by cells of the species Burkholderia pseudomallei.

5. The exopolysaccharide of claim 4 synthesised by cells of the Burkholderia pseudomallei strain NCTC 7431.

6. The exopolysaccharide of any one of claims 1 to 5, which has a molecular mass of more than 150 kd when determined by gel filtration in the presence of detergent.

7. The exopolysaccharide of any one of claims 1 to 3 which is synthesised by cells of the species Burkholderia mallei.

8. A method for the preparation of exopolysaccharides synthesised by cells of the bacterial species Burkholderia pseudomallei or Burkholderia mallei comprising the mechanical separation of the exopolysaccharide from the cell surface, followed by repetitive ethanol precipitation and affinity chromatography using the monoclonal antibody Mab 3015 produced by hybridoma DSM ACC 2218.

9. The method of claim 8 comprising the following steps:
(i) growing cells of Burkholderia pseudomallei or of Burkholderia mallei;
(ii) vigorous stirring of a cell suspension of said bacterial cells and removing bacterial cells by centrifugation;
(iii) heat-inactivation of the resulting supernatant followed by centrifugation;
(iv) subjection of the resulting supernatant to a cold 80% ethanol precipitation;
(v) dissolving of the precipitate and treatment with nucleases followed by heat-inactivation and centrifugation;
(vi) subjection of the resulting supernatant to a second ethanol precipitation; and
(vii) dissolving of the resulting precipitate followed by affinity chromatography on an affinity column containing monoclonal antibody Mab 3015 produced by hybridoma DSM ACC 2218.

10. An antibody recognising the exopolysaccharides of cells of the bacterial species Burkholderia pseudomallei and Burkholderia mallei.

11. Monoclonal antibody Mab 3015 produced by hybridoma DSM ACC 2218.

12. An antibody which specifically recognises the exopolysaccharide synthesised by cells of the bacterial species Burkholderia pseudomallei, wherein said exopolysaccharide is recognised by monoclonal antibody Mab 3015 produced by hybridoma DSM ACC 2218.

13. An antibody which specifically recognises the exopolysaccharide synthesised by cells of the bacterial species Burkholderia mallei, wherein said exopolysaccharide is recognised by monoclonal antibody Mab 3015 produced by hybridoma DSM ACC 2218.

14. The antibody of claims 10, 12 or 13 which is a monoclonal antibody, polyclonal antibody, chimeric antibody, (semi)synthetic antibody, antibody fragment (preferably the Fv, Fab or F(ab)₂ fragment) or a chemically modified derivative thereof.

15. The antibody of any one of claims 10 or 12 to 14, which is of the IgG1 isotype.

16. A continuous, stable antibody producing cell line which produces an antibody or antibody fragment according to any one of claims 10 to 15 or a derivative thereof.

17. The cell line of claim 16, which is a hybridoma cell line.

18. Hybridoma cell line DSM ACC 2218.

19. A method for the preparation of antibodies, fragments or derivatives thereof, of any one of claims 10 to 15, which comprises culturing a cell line of any one of claims 16 to 18 and isolating said antibody, antibody fragment or derivative thereof.

20. A method for the in vitro diagnosis of infection by Burkholderia pseudomallei, wherein samples of body fluids or tissue are tested for their reactivity with antibodies of claim 12 or fragments or derivatives thereof.

21. A method for the in vitro diagnosis of infection by Burkholderia mallei, wherein samples of body fluids or tissue are tested for their reactivity with antibodies of claim 13 or fragments or derivatives thereof.

22. A kit for the in vitro diagnosis of infection by Burkholderia pseudomallei comprising an antibody of claim 12 or a fragment or derivatives thereof and, optionally, further reagents conventionally used in immunodiagnostic methods.

23. A kit for the in vitro diagnosis of infection by Burkholderia mallei comprising an antibody of claim 13 or a fragment or derivatives thereof and, optionally, further reagents conventionally used in immunodiagnostic methods.

24. A method for the in vitro detection of antibodies directed against cells of the bacterial species Burkholderia pseudomallei in samples of body fluids or tissue, wherein said samples are tested for their reactivity with an exopolysaccharide of any one of claims 4 to 6.

25. A method for the in vitro detection of antibodies directed against cells of the bacterial species Burkholderia mallei in samples of body fluids or tissue, wherein said samples are tested for their reactivity with an exopolysaccharide of claim 7.

26. A kit for the in vitro detection of antibodies directed against cells of the bacterial species Burkholderia pseudomallei comprising an exopolysaccharide of any one of claims 4 to 6.

27. A kit for the in vitro detection of antibodies directed against cells of the bacterial species Burkholderia mallei comprising an exopolysaccharide of claim 7.

28. Vaccine comprising an exopolysaccharide of any one of claims 4 to 6 and optionally a pharmaceutically acceptable carrier.

29. Vaccine comprising an exopolysaccharide of claim 7 and optionally a pharmaceutically acceptable carrier.

30. Vaccine comprising an exopolysaccharide of any one of claims 4 to 6 and an exopolysaccharide of claim 7 and optionally a pharmaceutically acceptable carrier.

31. Vaccine comprising an antibody of claim 12 and optionally a pharmaceutically acceptable carrier.

32. Vaccine comprising an antibody of claim 13 and optionally a pharmaceutically acceptable carrier.

33. Vaccine comprising an antibody of claim 12 and an antibody of claim 13 and optionally a pharmaceutically acceptable carrier.

34. Vaccine comprising an antibody of claim 10 and optionally a pharmaceutically acceptable carrier.
